# EUROPEAN PATENT APPLICATION

(11) **EP 4 506 456 A1**
(43) Date of publication of application: **12.02.2025**
(21) Application number: 24193206.0
(22) Date of filing: 06.08.2024
(51) Int. Cl.: C12N 15/11, C12N 15/00, C12N 15/09, C12N 15/113, C07H 21/02

(54) **NOVEL OXO-RNA COMPOSITIONS AND THE RELATED APPLICATIONS THEREOF**

(30) Priority: 06.08.2023 US 202363531004 P
(71) Applicant: Lin, Shi-Lung, Arcadia, CA 91006 (US); Lin, Sam, Arcadia, CA 91006 (US); Hung, Wei-Chih, Tapei (TW); Liu, Hsien-Lin, Taoyuan City 330007 (TW); Wu, Yu-Jing, Taoyuan City 333001 (TW)
(72) Inventor: Lin, Shi-Lung, Arcadia, CA 91006 (US); Lin, Sam, Arcadia, CA 91006 (US); Hung, Wei-Chih, Tapei (TW); Liu, Hsien-Lin, Taoyuan City 330007 (TW); Wu, Yu-Jing, Taoyuan City 333001 (TW)
(74) Representative: Patentwerk B.V.

(57) **Abstract**

This invention relates to a novel modified RNA composition (called oxo-RNA) comprising at least an oxo-nucleotide (containing oxopurine) in its 3'-end region (*e.g.* 3'-tail, 3'-UTR). The oxo-nucleotide includes 8-hydroxyguanine/8-oxoguanine/7,8-dihydro-8-oxoguanine (or called oxo-G/oxo-dG) and 8-hydroxyadenine/8-oxoadenine (or called oxo-A/oxo-dA). Oxo-RNA can be a single-stranded RNA sequence or double-stranded duplex, or even an RNA-DNA hybrid duplex. Advantageously, this new oxo-RNA composition not only greatly enhance RNA/DNA stability and functionality but also can prevent TREX1-mediated degradation and the related non-specific immunity over-activation (*e.g.* cytokine storm). Most importantly, the constructs of oxo-RNA can be designed to mimic antisense RNA oligonucleotide (aRNA-ASO), small interfering RNA (siRNA), short hairpin RNA (shRNA), microRNA (miRNA) mimic, microRNA precursor (pre-miRNA), double-stranded RNA (dsRNA), RNA-DNA hybrid, long noncoding RNA (lncRNA), small activating RNA (saRNA), messenger RNA (mRNA), and/or self-amplifying RNA/mRNA (saRNA/samRNA), or a combination thereof.

## Description

### PRIORITY:

The present invention claims priority to U.S. Provisional Patent Application No. 63/531,004 filed on August 6, 2023, which is entitled "Novel In-Cell Transcription (ICT) Composition and the Related Vaccine Medicine Designs Thereof. The present invention also claims priority to U.S. Patent Application No. 17/489,357 filed on September 29, 2021, which is entitled "Novel mRNA Composition and Production Method for Use in Anti-Viral and Anti-Cancer Vaccines". Moreover, the present application is a continuation-in-part (CIP) application of the U.S. Patent Application No. 17/489,357 filed on September 29, 2021, which is entitled "Novel mRNA Composition and Production Method for Use in Anti-Viral and Anti-Cancer Vaccines". Furthermore, the present application is a continuation-in-part (CIP) application of the U.S. Patent Application No. 18/378,088 filed on October 9, 2023, which is entitled "Novel Induced Cytoplasmic IVT (ICIVT)-like Composition and the Related Vaccine Medicine Designs Thereof. All aforementioned prior patent applications are hereby incorporated by reference as if fully set forth herein.

### FIELD OF INVENTION:

This invention relates to a kind of novel modified RNA compositions containing at least an oxo-nucleotide which contains oxopurine, such as 8-hydroxyguanine/8-oxoguanine/7,8-dihydro-8-oxoguanine (oxo-G/oxo-dG) and 8-hydroxyadenine/8-oxoadenine (oxo-A/oxo-dA), in the sequences. This new kind of modified RNA compositions in either single-strand or double-strand conformation, or a combination thereof, is called "oxo-RNA". Preferably, the oxo-nucleotides are added in the 3'-end region *(e.g.* 3'-tail, 3'-UTR) of the oxo-RNA sequences in order to prevent TREX1-mediated degradation, no-go decay (NGD) as well as the related non-specific immunity over-activation (e.g. cytokine storm). The constructs of the oxo-RNA can be designed to mimic antisense RNA oligonucleotide (aRNA-ASO), small interfering RNA (siRNA), short hairpin RNA (shRNA), microRNA (miRNA), microRNA precursor (pri-/pre-miRNA), double-stranded RNA (dsRNA), RNA-DNA hybrid, long noncoding RNA (lncRNA), small activating RNA (saRNA), messenger RNA (mRNA), and/or self-amplifying RNA/mRNA (saRNA/samRNA), or a combination thereof. For facilitating delivery/transfection into the cells of interest in vitro, ex vivo as well as in vivo, the oxo-RNA composition can be mixed, conjugated, encapsulated or formulated with at least a reagent selected from, but not limited to, liposomes, nanoparticles, liposomal nanoparticles (LNP), multivesicular body (MVB) agents, exosomes, triglycylglycerin (TGG)-derived agents, sugar-/glucosamine-/galatosamine-based conjugating molecules, infusion agents, gene gun materials, electroporation agents, and/or transposons/retrotransposons, or a combination thereof. After delivery/transfection into the target cells of interest, the designed oxo-RNA may be used as vaccines and/or medicines for treating a variety of human diseases, including but not limited to Alzheimer's disease (AD), Parkinson's disease (PD), Spinal Muscular Atrophy (SMA) disease, motor neuron disease (MND), stroke, diabetes, myocardial infarction, hemophilia, anemia, leukemia, and many kinds of cancers as well as all sorts of viral and bacterial infections.

### BACKGROUND:

Oxo-nucleotides *(i.e.* containing oxopurine), such as containing 8-hydroxyguanine (= 8-oxoguanine, *e.g.* oxo-G/oxo-dG) and 8-hydroxyadenine (= 8-oxoadenine, *e.g.* oxo-A/oxo-dA), are a kind of oxidized purines frequently observed in cancer cells, particularly skin cancer cells. It has been shown that oxo-nucleotides presented in the middle of a DNA/RNA sequence can cause mismatch mutations, such as G::C to oxo-G::A pairing mutation. Also, oxo-nucleotides can cause accumulation of stalled ribosomes during mRNA translation and thus result in no-go decay (NGD) of mRNA (Boo and Kim, Exp. Mol. Med. 52:400-408, 2020). Hence, an ordinary skill person in the art will not anticipate the use of any oxo-nucleotide in the design of DNA/RNA vaccines or medicines. However, the present invention for the first time discloses a novel design of 3'-end oxo-nucleotide-tailed RNA compositions for preventing TREX1-mediated degradation, NGD as well as the related non-specific immunity over-activation (e.g. cytokine storm). In the present design, because the oxo-nucleotides are preferably added in the 3' -end region *(e.g.* 3'-tail, 3'-UTR) of desired DNA/RNA construct sequences, there is no concern about mismatch mutation or ribosome stalling problems.

Three prime repair exonuclease 1 (TREX1) is the most abundant 3'-to-5' exonuclease that degrades single-stranded and double-stranded DNA (ssDNA/dsDNA), single-stranded RNA as well as DNA-RNA hybrid sequences in mammalian cells (Yuan et al., J. Biol. Chem. 290:13344-13353, 2015). To overcome this problem while using RNA-/DNA-based molecules as vaccines and/or medicines, our priority invention of Induced Cytoplasmic IVT (ICIVT)-like Composition (U.S. Patent Application No. 18/378,088 to Lin et al.) has disclosed the utilization of 3'-end oxo-nucleotide-tailed DNA and mRNA for preventing TREX1-mediated degradation as well as the related non-specific immune response activation. Yet, the description of using oxo-nucleotides in the designs of various modified non-coding RNA (ncRNA) constructs, including but not limited to antisense RNA oligonucleotide (aRNA-ASO), small interfering RNA (siRNA), short hairpin RNA (shRNA), double-stranded RNA (dsRNA), small activating RNA (saRNA), and microRNA (miRNA)/microRNA precursor (pre-miRNA), are still not fully revealed. Hence, the present invention is aimed to address the novel designs and related utilizations of these oxo-nucleotide-modified ncRNAs, called oxo-ncRNA.

### SUMMARY OF THE INVENTION:

The principle of the present invention is relied on the novel composition designs described in the claimed priority U.S. Patent Application No. 18/378,088 (to Lin et al.). By incorporating at least an oxo-nucleotide (containing oxopurine), such as containing 8-hydroxyguanine/8-oxoguanine (oxo-G/oxo-dG) and/or 8-hydroxyadenine/8-oxoadenine (oxo-A/oxo-dA), in the 3'-end *(e.g.* 3'-tail, 3'-UTR) of a designed RNA/DNA construct, the resulting oxo-RNA/oxo-DNA composition so obtained can bypass TREX1-mediated fast degradation, so as to deliver the full functional specificity and potency of the designed RNA/DNA in the cells of interest without concern for RNA/DNA stability or non-specific immunity over-activation problems. Notably, oxo-nucleotide-modified RNA (oxo-RNA) can further bypass the cellular cGAS-STING pathway, preventing any possible over-activation of pro-inflammatory cytokine responses or called cytokine storm. Obviously, this key novelty is an important breakthrough for the safety use of related RNA-based vaccines and medicines. Till today, fast degradation and non-specific immune response activation are still two of the major problems greatly hindering the clinic use of RNA-/DNA-based platform constructs as vaccines or medicines, significantly limiting the usefulness of small non-coding RNA (e.g. siRNA, shRNA and miRNA) as well as long protein-/peptide-coding mRNA platform constructs.

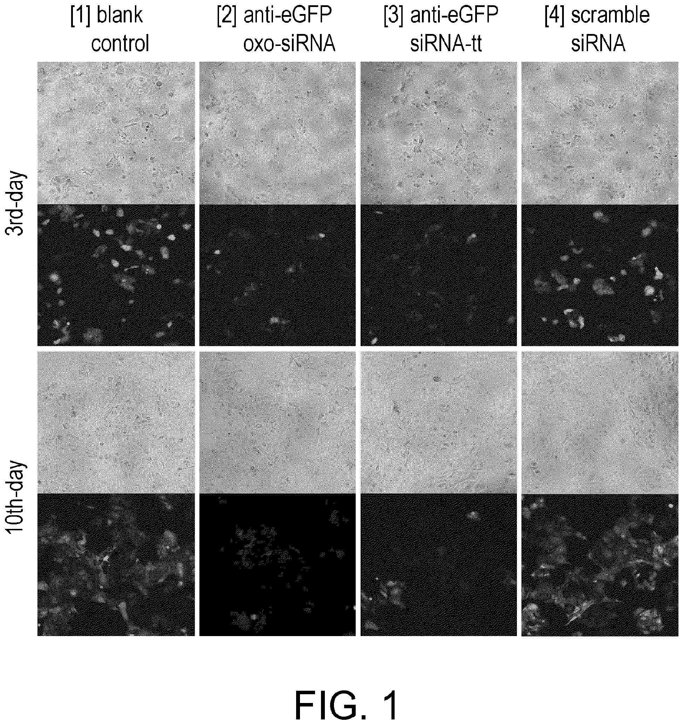

Due to the tight regulation of RNA-Induced Silencing Complex (RISC) formation during RNA interference (RNAi)-mediated gene silencing events, some previously reported nucleotide modifications have been shown to offer no significant advantage over conventional siRNA or shRNA designs. For example, pseudouridine modification has been reported to provide lower potency if added in the 5'-end or middle region of siRNA, while showing limited significance if added in the 3'-end. Also, in order to increase the thermodynamic stability of siRNA duplex constructs, two thymidine nucleotides (called TT-overhangs) are frequently added in the 3'-ends of conventional siRNA, which however cause TREX1-mediated degradation and thus greatly reduce the resulting gene silencing effects in cells. To solve this problem, the present invention for the first time discloses that the use of 3'-end oxo-nucleotide (such as containing *dG)-tailed siRNA and shRNA constructs (namely oxo-siRNA and oxo-shRNA, respectively) can form effective RISCs in cells, so as to efficiently silence the targeted gene expressions of the designed siRNA and/or shRNA without loss of its target specificity or knockdown potency (FIG. 1).

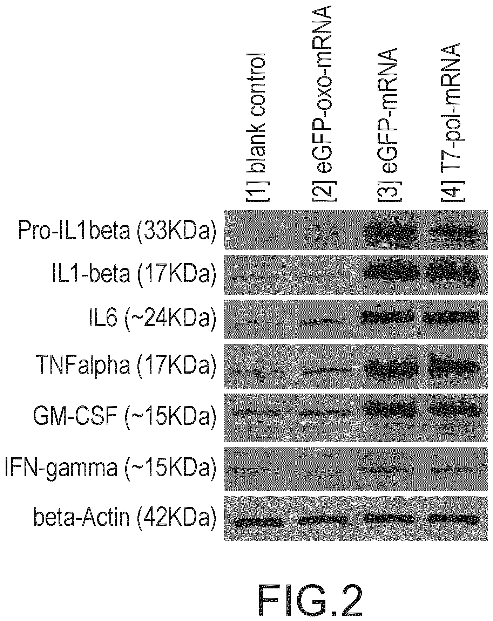

Moreover, the invented 3'-end oxo-nucleotide-tailed RNA (oxo-RNA) compositions not only can prevent TREX1-mediated degradation but also strongly inhibit possible non-specific immunity over-activation, particularly markedly diminishing the stimulated expression of key pro-inflammatory cytokines/factors, such as IL-1beta, IL-6, TNFalpha, interferon gamma, and GM-CSF (FIG. 2). This finding clearly indicates the importance and urgent need of using oxo-siRNA/oxo-shRNA in place of conventional siRNA/shRNA constructs for developing novel RNA interference (RNAi)-based medicines and/or therapies, so as to overcome the unsolved clinical problem of non-specific immunity over-activation in particular, cytokine storm.

In one preferred embodiment, the designed oxo-RNA construct is an RNA duplex, such as siRNA, dsRNA and saRNA, containing at least an oxo-nucleotide modification in the 3'-ends of both strands of the RNA duplex equences, so as to protect both strands from RNA degradation. As a result, this dual strand oxo-nucleotide modification design can provide the best protection effect for siRNA, dsRNA and saRNA constructs against not only TREX1-mediated but also many other RNase-induced degradation.

In another preferred embodiment, the designed oxo-RNA construct contains at least an oxo-nucleotide modification in only one strand of the duplex structures of siRNA, dsRNA and/or saRNA, so as to preferentially protect one desired strand while making the other strand degraded. This approach provides a great advantage for preferentially selecting the desired RNA strand (in the form of oxo-RNA) for RISC or RITA complex formation, leading to much higher functional potency and specificity as well as lower off-target side effects. Notably, this partial oxo-RNA modification design clearly is a whole new RNA platform construct useful for solving the non-specific strand selection problem of conventional siRNA, dsRNA and saRNA.

In the third preferred embodiment, the designed oxo-RNA construct is single-strand RNA (ssRNA), such as aRNA-ASO, shRNA, miRNA, pri-/pre-miRNA and mRNA/saRNA/samRNA, which contains at least an oxo-nucleotide in its 3'-end region, so as to protect the RNA sequence from any possible 3'-to-5' exonuclease degradation. Noteworthily, this kind of single-strand oxo-RNA compositions has been shown to markedly enhance the potency and duration of RNA functionality, such as mRNA translation, as described in our priority U.S. Patent Application No. 18/378,088 (to Lin et al.).

To further enhance oxo-RNA stability, the U (uridine/uracil) content of oxo-RNA may be completely or partially replaced by at least a modified U analog, such as pseudouridine (p), methyluridine (t/tm/um), methylpseudouridine (fm/m1f), methoxyuridine (mo5U), and/or other similar nucleotide analogs, or a combination thereof. Alternatively, the C (cytosine/cytidine) content of oxo-RNA may also be further completely or partially replaced by 5-methylcytidine (m⁵C) or N4-acetylcytidine (ac⁴C), or both. Conceivably, one oxo-RNA may contain both of U and C analog modifications. Hence, oxo-nucleotide modification may be combinedly used with other nucleotide modifications for further increasing the stability and potency of non-coding RNAs (e.g. siRNA, shRNA and/or miRNA) as well as protein-/peptide-coding mRNAs.

Our priority U.S. Patent Application No. 18/378,088 has disclosed a special new design of promoter-linked RNA/mRNA-coding DNA (PLRcD) construct useful for inducing IVT-like RNA/mRNA amplification in the cytoplasm of the transfected cells of interest, leading to an intracellular cytoplasmic RNA/mRNA expression system. The PLRcD construct is formed by inserting at least an internal ribosome entry site (IRES)-linked kozak motif (IRES-kozak) into a 5'-UTR region located between the promoter and RNA/mRNA-coding sequences. Also, in order to activate the encoded RNA/mRNA expression, the designed PLRcD construct must be co-transfected with at least a DNA-dependent RNA polymerase (DdRP) mRNA, including but not limited to T7, T3, and/or SP6 DdRP mRNA. Notably, for preventing non-specific interferon-associated immunity activation as well as TREX1-mediated degradation , the designed PLRcD construct is dephosphorylated in the 5'-end and tailed by at least a 8-hydroxyguanine/8-oxoguanine (8-OHG/8-oxo-G) and/or its derived nucleotide analog, particularly 8-hydroxy-2-deoxyguanosine (8-OHdG/8-oxo-dG), in the 3'-end, respectively, while the DdRP mRNA is 5'-capped by m7G(5')ppp(5')N (m7G, cap-0) and/or its related cap *(i.e.* cap-1 and/or cap-2) analogs and may be further tailed by at least a modified nucleotide analog *(e.g.* 3'-cap modified G/U/A molecule, such as 8-oxo-G/dG, 8-oxo-A/dA and/or 5'-phosphorothioate-U) in the 3'-end region, respectively.

For 5'-cap incorporation, the capping enzymes include, but not limited to, coronaviral NSP12, NSP9/14, NSP10/16, and/or vaccinia capping enzyme as well as 2'-O-methyltransferase, or a combination thereof. For 3'-oxo-nucleotide (or previously called 3'-cap) incorporation, terminal deoxynucleotidyl transferases (TdT), terminal uridylyl transferases and/or polymerase theta are preferably used. Alternatively, machine synthesis using oligonucleotide synthesizer is another preferred method to incorporate oxo-nucleotides into the 5'-/3'-ends of RNA/DNA. Also, for further preventing non-specific immunity over-activation, the nucleotide contents of oxo-RNA/DNA may be further completely or partially modified by another kind of modified nucleotides, such as pseudouridine, methyluridine, methylpseudouridine, methoxyuridine, methylcytidine, acetylcytidine, and/or any other similar nucleotide analog, or a combination thereof.

The kozak motif is a nucleotide sequence of 5'-RCCRCC-3' (SEQ ID NO:1; R is A or G) or 5'-RCCDCC-3' (SEQ ID NO:2; D is A, G or T/U), while the IRES may contain at least one homologue of the following hairpin/stem-loop-containing sequences, of which the contents U (uracil/uridine) and T (thymine/thymidine) are exchangable, including but not limited to:
(1) 
5'-GCUCCCUUCA ACUUUAACAU GGAAGUGCUU UCUGUGACUU
UAAAAGUAAG UGCUUCCAUG UUUUAGUAGG AGU-3'    (73-nt)
(SEQ ID NO:3)
(2) 
5'-CCUUUGCUUU AACAUGGGGG UACCUGCUGU GUGAAACAAA
AGUAAGUGCU UCCAUGUUUC AGUGGAGG-3'    (68-nt)    (SEQ ID
NO:4)
(3) 
5'-CCACCACUUA AACGUGGAUG UACUUGCUUU GAAACUAAAG
AAGUAAGUGC UUCCAUGUUU UGGUGAUGG-3'    (69-nt)    (SEQ ID
NO:5)
(4) 
5'-CCUCUACUUU AACAUGGAGG CACUUGCUGU GACAUGACAA
AAAUAAGUGC UUCCAUGUUU GAGUGUGG-3'    (68-nt)    (SEQ ID
NO:6)
(5) 
5'-CUGUGUGGCU GUCACUCGGC UGCAUGCUUA GUGCACUCAC
GCAG-3' (44-nt) (SEQ ID NO:7)
(6) 
5'-CUGUGUGGCU GUCACUCGGC UGCAUGCUUA GUGCACUCAC
GCAGUAUAAU UAAUAACUAA UUACU-3'    (65-nt)    (SEQ ID NO:8)
(7) 
5'-GUCGUUGACA GGACACGAGU AACUCGUCUA UCUUCUGCAG
GCUGCUUACG GUUUCGUCCG UGUUGCAGCC GAUCAUCAGC
 
ACAUCUAGGU UUCGUCCGGG UGUGACCGAA AGGUAAGAUG
GAGAGCCUUG UCCCUGGUUU CAACGAG-3’ (147-nt) (SEQ ID
NO:9)
(8) 
5'-AAUUAUAAAU UACCAGAUGA UUUUACAGGC UGCGUUAUAG
CUUGGAAUUC UAACAAUCUU GAUUCUAAGG UUGGUGGUAA
UUAUAAUU-3'    (88-nt)    (SEQ ID NO:10)
(9) 
5'-CACAAAUAUU ACCAGAUCCA UCAAAACCAA GCAAGAGGUC
AUUUAUUGAA GAUCUACUUU UCAACAAAGU GACACUUGCA
GAUGCUGGCU UCAUCAAACA AUAUGGUGAU UGCCUUGGUG
AUAUUGCUG-3'    (129-nt)    (SEQ ID NO:11)
(10) 
5'-GCAAAAAUGU GAUCUUGCUU GUAAAUACAA UUUUGAGAGG
UUAAUAAAUU ACAAGUAGUG CUAUUUUUGU AUUUAGGUUA
GCUAUUUAGC UUUACGUUCC AGGAUGCCUA GUGGCAGCCC
CACAAUAUCC AGGAAGCCCU CUCUGCGGUU UUUCAGAUUC
GUUAGUCGAA AAACCUAAGA AAUUUAAUG-3'    (189-nt)    (SEQ ID
NO:12)
(11) 
5'-CACUCCCCUG UGAGGACUAC UGUCUUCACG CAGAAAGCGU
CUAGCCAUGG CGUUAGUAUG AGUGUCGUGC AGCCUCCAGG
ACCCCCCCUC CCGGGAGAGC CAUAGUGGUC UGCGGAACCG
GUGAGUACAC CGGAAUUGCC AGGACGACCG GGUCCUUUCU
UGGAUCAACC CGCUCAAUGC CUGGAGAUUU GGGCGUGCCC
CCGCGAGACU GCUAGCCGAG UAGUGUUGGG UCGCGAAAGG
CCUUGUGGUA CUGCCUGAUG GGUGCUUGCG AGUGCCCCGG
GAGGUCUCGU AGAC-3'    (294-nt)    (SEQ ID NO:13)
(12) 
5'-GGACACGAGU AACUCGUCUA UCUUCUGCAG GCUGCUUACG
GUUUCGUCCG UGUUG-3' (55-nt) (SEQ ID NO:14)
(13)

5'-CAGCCGAUCA UCAGCACAUC UAGGUUUUGU CCGGGUGUGA
CCGAAAGGUA AG-3' (52-nt) (SEQ ID NO:15)

For performing induced cytoplasmic IVT-like transcription (ICIVT), our claimed priority U.S. Patent Application No. 18/378,088 has further revealed a novel method of co-transfecting at least a designed PLRcD template along with at least a DdRP mRNA (e.g. T7, T3, and/or SP6 DdRP mRNA) into the target cells of interest. Also, since the newly transcribed RNA/mRNA products so obtained (located in cytoplasm/cytosol) may not carry any proper 5'-cap structure, the invented IRES-kozak motif in the designed PLRcD template will then serve as an internal ribosome entry site (IRES) for facilitating ribosome assembly on the transcribed mRNAs, so as to activate protein/peptide synthesis. Meanwhile, the IRES-linked kozak motif also marks the exact start location of mRNA translation, leading to highly precise and efficient protein/peptide synthesis. Yet, in addition to protein-/peptide-coding mRNA, the PLRcD-encoded RNA may also be non-coding RNA (ncRNA), such as antisense RNA oligonucleotide (aRNA-ASO), small interfering RNA (siRNA), short hairpin RNA (shRNA), small activating RNA (saRNA), and/or microRNA (miRNA)/microRNA precursor (pre-miRNA), all of which do not need the IRES-kozak motif for their functionings.

IRES is a highly structured nucleotide sequence, frequently containing harpin-like and/or stem-loop structures. For producing highly structured PLRcD templates, our another priority U.S. patent application No. 17/489,357 (`357) has developed a novel PCR-IVT methodology, using a specific helicase activity for overcoming the low efficiency problem of highly structured RNA amplification in traditional IVT reactions. Because hairpin-like stem-loop structures may resemble intrinsic transcription termination signals of prokaryotic RNA polymerases (McDowell et al, Science 266:822-825, 1994), the `357 method adopts a new IVT system with a mixture of RNA polymerase and helicase activities to overcome this problem. As a result, the additional helicase activity in IVT markedly reduces the secondary structures of designed PLRcD templates and the resulting RNA/mRNA products, leading to high throughput RNA/mRNA amplification. Based on our studies, coronaviral NSP7 and NSP13 proteins are two of the identified helicases useful for enhancing the amplification efficiency of IVT and ICIVT.

For facilitating delivery/transfection of the designed oxo-RNA construct(s) into the target cells of interest in vitro, ex vivo and/or in vivo, the designed oxo-RNA composition can be mixed, conjugated, encapsulated and/or formulated with at least a delivery/transfection agent selected from, but not limited to, liposomes, nanoparticles, liposomal nanoparticles (LNP), exosomes, sugar-/glucosamine-/galatosamine-based conjugating molecules, infusion/transfusion agents, glycylglycerin-derived molecules, gene gun materials, electroporation agents, and/or transposons/retrotransposons, or a combination thereof.

### A. Definitions

To facilitate understanding of the invention, a number of terms are defined below:
Nucleic Acid: a polymer of deoxyribonucleic acid (DNA) or ribonucleic acid (RNA), either single or double stranded.
Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. A nucleoside containing at least one phosphate group bonded to the 3' or 5' position of the pentose is a nucleotide. DNA and RNA are consisted of different types of nucleotide units called deoxyribonucleotide and ribonucleotide, respectively.
Deoxyribonucleoside Triphosphates (dNTPs): the building block molecules for DNA synthesis, including dATP, dGTP, dCTP, and dTTP and sometimes may further containing some modified deoxyribonucleotide analogs.
Ribonucleoside Triphosphates (rNTPs): the building block molecules for RNA synthesis, including ATP, GTP, CTP, and UTP and sometimes may further containing pseudouridine, 5'methyluridine, methoxyuridine, and/or some other modified ribonucleotide analogs.
Nucleotide Analog: a purine or pyrimidine nucleotide that differs structurally from adenine (A), thymine (T), guanine (G), cytosine (C), or uracil (U), but is sufficiently similar to substitute for the normal nucleotide in a nucleic acid molecule.
Oligonucleotide: a molecule comprised of two or more monomeric units of DNA and/or RNA, preferably more than three, and usually more than ten. An oligonucleotide longer than 13 nucleotide monomers is also called polynucleotiude. The exact size will depend on many factors, which in turn depends on the ultimate function or use of the oligonucleotide. The oligonucleotide may be generated in any manner, including chemical synthesis, DNA replication, RNA transcription, reverse transcription, or a combination thereof.
Nucleic Acid Composition: a nucleic acid composition refers to an oligonucleotide or polynucleotide such as a DNA or RNA sequence, or a mixed DNA/RNA sequence, in either a single-stranded or a double-stranded molecular structure.
Gene: a nucleic acid composition whose oligonucleotide or polynucleotide sequence codes for an RNA and/or a polypeptide (protein). A gene can be either RNA or DNA. A gene may encode a non-coding RNA, such as small hairpin RNA (shRNA), microRNA (miRNA), rRNA, tRNA, snoRNA, snRNA, and their RNA precursors as well as derivatives. Alternatively, a gene may encode a protein-coding RNA essential for protein/peptide synthesis, such as messenger RNA (mRNA) and its RNA precursors as well as derivatives. In some cases, a gene may encode a protein-coding RNA that also contains at least a microRNA or shRNA sequence.
Primary RNA Transcript: an RNA sequence that is directly transcribed from a gene without any RNA processing or modification.
Precursor messenger RNA (pre-mRNA): primary RNA transcripts of a protein-coding gene, which are produced by eukaryotic type-II RNA polymerase (Pol-II) machineries in eukaryotes through an intracellular mechanism termed transcription. A pre-mRNA sequence contains a 5'-untranslated region (UTR), a 3'-UTR, exons and introns.
Intron: a part or parts of a gene transcript sequence encoding non-protein-reading frames, such as in-frame intron, 5'-UTR and 3'-UTR.
Exon: a part or parts of a gene transcript sequence encoding protein-reading frames (cDNA), such as cDNA for cellular genes, growth factors, insulin, antibodies and their analogs/homologs as well as derivatives.
Messenger RNA (mRNA): assembly of pre-mRNA exons, which is formed after intron removal by intracellular RNA splicing machineries (e.g. spliceosomes) and served as a protein-coding RNA for peptide/protein synthesis. Structurally, mRNA sequence may comprise 5'-cap nucleotide [*e.g.* m7G(5')ppp(5')N-], 5'-untranslated region (5'-UTR), at least a Kozak consensus translation initiation site (*e.g.* 5'-GCCACC-3'), at least a protein/peptide-coding region, polyadenylation signals (*e.g.* 5'-AUAAA-3' or 5'-AUUAAA-3'), and/or 3' -UTR with or without poly(A) tail. The proteins/peptides encoded by mRNAs include, but not limited to, enzymes, growth factors, insulin, antibodies and their analogs/homologs as well as derivatives.
Complementary DNA (cDNA): a single-stranded or double-stranded DNA that contains a sequence complementary to an mRNA sequence and does not contain any intronic sequence.
Sense: a nucleic acid molecule in the same sequence order and composition as the homologous mRNA. The sense conformation is indicated with a "+", "s" or "sense" symbol.
Antisense: a nucleic acid molecule complementary to the respective mRNA molecule. The antisense conformation is indicated as a "-" symbol or with an "a" or "antisense" in front of the DNA or RNA, *e.g.*, "aDNA" or "aRNA".
Base Pair (bp): a partnership of adenine (A) with thymine (T), or of cytosine (C) with guanine (G) in a double stranded DNA molecule. In RNA, uracil (U) is substituted for thymine. Generally the partnership is achieved through hydrogen bonding. For example, a sense nucleotide sequence "5'-A-T-C-G-U-3'" can form complete base pairing with its antisense sequence "5'-A-C-G-A-T-3'".
5'-end: a terminus lacking a nucleotide at the 5' position of successive nucleotides in which the 5'-hydroxyl group of one nucleotide is joined to the 3'-hydroyl group of the next nucleotide by a phosphodiester linkage. Other groups, such as one or more phosphates, may be present on the terminus.
3'-end: a terminus lacking a nucleotide at the 3' position of successive nucleotides in which the 5'-hydroxyl group of one nucleotide is joined to the 3'-hydroyl group of the next nucleotide by a phosphodiester linkage. Other groups, often a hydroxyl group, may be present on the terminus. A 3'-UTR may be located near or next to the 3'-end of an oligonucleotide.
Template: a nucleic acid molecule being copied by a nucleic acid polymerase. A template can be single-stranded, double-stranded or partially double-stranded, RNA or DNA, depending on the polymerase. The synthesized copy is complementary to the template, or to at least one strand of a double-stranded or partially double-stranded template. Both RNA and DNA are synthesized in the 5' to 3' direction. The two strands of a nucleic acid duplex are always aligned so that the 5' ends of the two strands are at opposite ends of the duplex (and, by necessity, so then are the 3' ends).
Nucleic Acid Template: a double-stranded DNA molecule, double-stranded RNA molecule, hybrid molecules such as DNA-RNA or RNA-DNA hybrid, or single-stranded DNA or RNA molecule.
Conserved: a nucleotide sequence is conserved with respect to a pre-selected (referenced) sequence if it non-randomly hybridizes to an exact complement of the pre-selected sequence.
Homologous or Homology: a term indicating the similarity between a polynucleotide and a gene or mRNA sequence. A nucleic acid sequence may be partially or completely homologous to a particular gene or mRNA sequence, for example. Homology may be expressed as a percentage determined by the number of similar nucleotides over the total number of nucleotides.
Complementary or Complementarity or Complementation: a term used in reference to matched base pairing between two polynucleotides (e.g. sequences of an mRNA and a cDNA) related by the aforementioned "base pair (bp)" rules. For example, the sequence "5'-A-G-T-3'" is complementary to not only the sequence "5'-A-C-T-3'" but also to "5'-A-C-U-3'". Complementation can be between two DNA strands, a DNA and an RNA strand, or between two RNA strands. Complementarity may be "partial" or "complete" or "total". Partial complementarity or complementation occurs when only some of the nucleic acid bases are matched according to the base pairing rules. Complete or total complementarity or complementation occurs when the bases are completely or perfectly matched between the nucleic acid strands. The degree of complementarity between nucleic acid strands has significant effects on the efficiency and strength of hybridization between nucleic acid strands. This is of particular importance in amplification reactions, as well as in detection methods that depend on binding between nucleic acids. Percent complementarity or complementation refers to the number of mismatch bases over the total bases in one strand of the nucleic acid. Thus, a 50% complementation means that half of the bases were mismatched and half were matched. Two strands of nucleic acid can be complementary even though the two strands differ in the number of bases. In this situation, the complementation occurs between the portion of the longer strand corresponding to the bases on that strand that pair with the bases on the shorter strand.
Complementary Bases: nucleotides that normally pair up when DNA or RNA adopts a double stranded configuration.
Complementary Nucleotide Sequence: a sequence of nucleotides in a single-stranded molecule of DNA or RNA that is sufficiently complementary to that on another single strand to specifically hybridize between the two strands with consequent hydrogen bonding.
Hybridize and Hybridization: the formation of duplexes between nucleotide sequences which are sufficiently complementary to form complexes via base pairing. Where a primer (or splice template) "hybridizes" with target (template), such complexes (or hybrids) are sufficiently stable to serve the priming function required by a DNA polymerase to initiate DNA synthesis. There is a specific, *i.e.* non-random, interaction between two complementary polynucleotides that can be competitively inhibited.
Posttranscriptional Gene Silencing: a targeted gene knockout or knockdown effect at the level of mRNA degradation or translational suppression, which is usually triggered by either foreign/viral DNA or RNA transgenes or small inhibitory RNAs.
RNA Interference (RNAi): a posttranscriptional gene silencing mechanism in eukaryotes, which can be triggered by small inhibitory RNA molecules such as microRNA (miRNA), small hairpin RNA (shRNA) and small interfering RNA (siRNA). These small RNA molecules usually function as gene silencers, interfering with expression of intracellular genes containing either completely or partially complementarity to the small RNAs.
MicroRNA (miRNA): single-stranded RNAs capable of binding to targeted gene transcripts that have partial complementarity to the miRNA. MiRNA is usually about 17-27 oligonucleotides in length and is able to either directly degrade its intracellular mRNA target(s) or suppress the protein translation of its targeted mRNA, depending on the complementarity between the miRNA and its target mRNA. Natural miRNAs are found in almost all eukaryotes, functioning as a defense against viral infections and allowing regulation of gene expression during development of plants and animals.
Precursor MicroRNA (Pre-miRNA): hairpin-like single-stranded RNAs containing stem-arm and stem-loop regions for interacting with intracellular RNaseIII endoribonucleases to produce one or multiple microRNAs (miRNAs) capable of silencing a targeted gene or genes complementary to the microRNA sequence(s). The stem-arm of a pre-miRNA can form either a perfectly (100%) or a partially (mis-matched) hybrid duplexes, while the stem-loop connects one end of the stem-arm duplex to form a circle or hairpin-loop conformation. In the present invention, however, precursor of microRNA may also includes pri-miRNA.
Small interfering RNA (siRNA): short double-stranded RNAs sized about 18-27 perfectly base-paired ribonucleotide duplexes and capable of degrading target gene transcripts with almost perfect complementarity.
Small or short hairpin RNA (shRNA): single-stranded RNAs that contain a pair of partially or completely matched stem-arm nucleotide sequences divided by an unmatched loop or bubble oligonucleotide to form a hairpin-like structure. Many natural miRNAs are derived from small hairpin-like RNA precursors, namely precursor microRNA (pre-miRNA).
Vector: a recombinant nucleic acid composition such as recombinant DNA (rDNA) capable of movement and residence in different genetic environments. Generally, another nucleic acid is operatively linked therein. The vector can be capable of autonomous replication in a cell in which case the vector and the attached segment is replicated. One type of preferred vector is an episome, i.e., a nucleic acid molecule capable of extrachromosomal replication. Preferred vectors are those capable of autonomous replication and expression of nucleic acids. Vectors capable of directing the expression of genes encoding for one or more polypeptides and/or non-coding RNAs are referred to herein as "expression vectors" or "expression-competent vectors". Particularly important vectors allow cloning of cDNA from mRNAs produced using a reverse transcriptase. A vector may contain components consisting of a viral or a type-II RNA polymerase (Pol-II or pol-2) promoter, or both, a Kozak consensus translation initiation site (such as 5'-GCCACC-3'), polyadenylation signals (such as 5'-AUAAA-3' or 5'-AUUAAA-3'), a plurality of restriction/cloning sites, a pUC origin of replication, a SV40 early promoter for expressing at least an antibiotic resistance gene in replication-competent prokaryotic cells, an optional SV40 origin for replication in mammalian cells, and/or a tetracycline responsive element. The structure of a vector can be a linear or circular form of single- or double-stranded DNA selected form the group consisting of plasmid, viral vector, transposon, retrotransposon, DNA transgene, jumping gene, and a combination thereof.
Promoter: a nucleic acid to which a polymerase molecule recognizes, perhaps binds to, and initiates RNA transcription. For the purposes of the instant invention, a promoter can be a known polymerase binding site, an enhancer and the like, any sequence that can initiate synthesis of RNA transcripts by a desired polymerase.
RNA Processing: a cellular mechanism responsible for RNA maturation, modification and degradation, including RNA splicing, intron excision, exosome digestion, nonsense-mediated decay (NMD), RNA editing, RNA processing, 5'-capping, 3'-poly(A) tailing, and a combination thereof.
Gene Delivery: a genetic engineering method selected from the group consisting of polysomal transfection, liposomal transfection, chemical (nanoparticle) transfection, electroporation, viral infection, DNA recombination, transposon insertion, jumping gene insertion, microinjection, gene-gun penetration, and a combination thereof.
Genetic Engineering: a DNA recombination method selected from the group consisting of DNA restriction and ligation, homologous recombination, transgene incorporation, transposon insertion, jumping gene integration, retroviral infection, and a combination thereof.
Transfected Cell: a single or a plurality of eukaryotic cells after being artificially inserted with at least a nucleic acid sequence or protien/peptide molecule into the cell(s), selected from the group consisting of a somatic cell, a tissue cell, a stem cell, a germ-line cell, a tumor cell, a cancer cell, a virus-infected cell, and a combination thereof.
Antibody: a peptide or protein molecule having a pre-selected conserved domain structure coding for a receptor capable of binding a pre-selected ligand.
Pharmaceutical and/or therapeutic Application: a biomedical utilization and/or apparatus useful for stem cell generation, drug/vaccine development, non-transgenic gene therapy, cancer therapy, disease treatment, wound healing, tissue/organ repair and regeneration, and high-yield production of proteins/peptides/antibodies, drug ingredients, medicines, vaccines and/or food supplies, and a combination thereof.

### B. Compositions and Applications

A novel modified RNA composition comprising at least an oxo-nucleotide (containing oxopurine), including but not limited to containing 8-hydroxyguanine/8-oxoguanine/7,8-dihydro-8-oxoguanine (oxo-G/oxo-dG) and 8-hydroxyadenine/8-oxoadenine (oxo-A/oxo-dA), in the 3'-end region (such as 3'-tail, 3'-UTR) of its single-stranded or double-stranded sequence, or a combination thereof. This new kind of 3'-end oxo-nucleotide-tailed RNA compositions, called oxo-RNA, can be used to prevent TREX1-mediated degradation, no-go decay (NGD) as well as the related non-specific immunity over-activation (e.g. cytokine storm). The conformation constructs of oxo-RNA can be designed to mimic antisense RNA oligonucleotide (aRNA-ASO), small interfering RNA (siRNA), short hairpin RNA (shRNA), microRNA (miRNA), microRNA precursor (pri-/pre-miRNA), double-stranded RNA (dsRNA), RNA-DNA hybrid, long noncoding RNA (lncRNA), small activating RNA (saRNA), messenger RNA (mRNA), and/or self-amplifying RNA/mRNA (saRNA/ samRNA), or a combination thereof.

For delivery/transfection into the cells of interest in vitro, ex vivo and/or in vivo, the oxo-RNA composition may be further mixed, conjugated, encapsulated or formulated with at least a reagent selected from, but not limited to, liposomes, nanoparticles, liposomal nanoparticles (LNP), exosomes, triglycylglycerin (TGG)-derived agents, sugar-/glucosamine-/galatosamine-based conjugating molecules, infusion agents, gene gun materials, electroporation agents, and/or transposons/retrotransposons, or a combination thereof. After delivery/transfection into the target cells of interest, the oxo-RNA can be used as vaccines and/or medicines for treating a variety of human diseases, including but not limited to Alzheimer's disease (AD), Parkinson's disease (PD), Spinal Muscular Atrophy (SMA) disease, motor neuron disease (MND), stroke, diabetes, myocardial infarction, hemophilia, anemia, leukemia, and many kinds of cancers as well as all sorts of viral and bacterial infections

### BRIEF DESCRIPTION OF THE DRAWINGS:

Referring particularly to the drawings for the purpose of illustration only and not limitation, there is illustrated:
FIG. 1 shows the siRNA-mediated gene silencing effects against copGFP expression in copGFP-expressing A549 cells. From left to right, the panels show [1] blank control (without siRNA addition), [2] one treatment of siRNA with additional 3'-oxo-G/oxo-dG (*dG) tail (1-2000 nM oxo-siRNA; SEQ ID NO:22), [3] one treatment of conventional siRNA with 3'-TT-overhang (1-2000 nM siRNA-tt; SEQ ID NO:23), and [4] one treatment of scramble siRNA (1-2000 nM) without any nucleotide overhang or tail (negative control; SEQ ID NO:24). These microscopic examination results clearly demonstrate a very high gene silencing effect (>85% knockdown rate) in both of the test groups using conventional siRNA-tt and the newly invented oxo-siRNA compared to that of the blank and negative control groups. Notably, the oxo-siRNA-mediated gene silencing effect (>12-day duration) lasts significantly longer than that of using conventional siRNA-tt (8~10-day duration).
FIG. 2 shows Western blotting results of the protein expression of key pro-inflammatory cytokines/factors, such as IL-1ß, IL-6, TNFalpha, IFNgamma and GM-CSF, induced by [1] blank control (without any RNA addition), [2] one treatment of oxo-RNA (eGFP-mRNA with a 3'-oxo-dG-modified poly-A tail), [3] one treatment of eGFP-coding mRNA with a unmodified 3'-poly-A tail, and [4] one treatment of T7-polymerase-coding mRNA with a unmodified 3'-poly-A tail, respectively, in the transfected co-culture cell lines of Jurkat T and THP-1 cells.

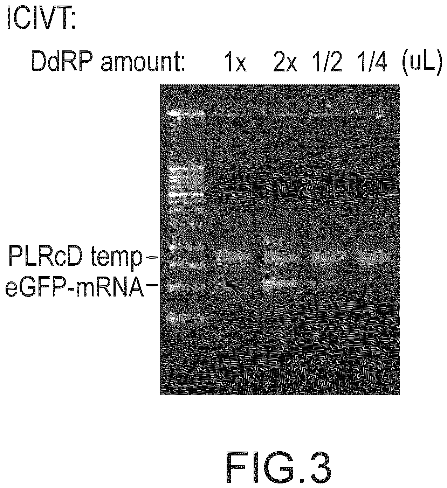

FIG. 3 shows the nondenaturing agarose gel electrophoresis results of IVT-like reactions using the mixture composition of a designed PLRcD template (e.g. PLRcD dsDNA encoding green fluorescent eGFP mRNA) and T7 DdRP. Notably, the amplification level of PLRcD-encoded mRNA (e.g. eGFP mRNA) is proportionally regulated by the used amount of DdRP.

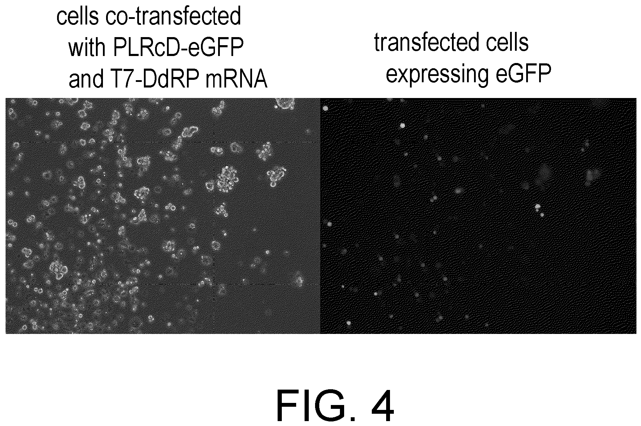

FIG. 4 shows microscopic examination results of the resulting eGFP protein expression induced by LNP-mediated co-transfection of the mixture composition of the designed PLRcD template (from FIG. 3) and T7 DdRP oxo-mRNA into the targeted cells of interest.

### EXAMPLES:

### 1. Human Cell Isolation, Cultivation and Treatments

Human cancer and normal cell lines, such as MCF7, HepG2, Jurkat T, THP-1, copGFP-positive A549 and BEAS-2B cells, were obtained either from the American Type Culture Collection (ATCC, Rockville, MD) or our collaborators and then maintained according to individual manufacturer's suggestions. In some cases in order to prevent dose dilution by cell division, cytochalasin D (final concentration = 0.25 µM) is added in each cell culture after treatments. For intracellular mRNA transfection, 0.5-1000 microgram (µg) of the mixture composition containing both PLRcD template and DdRP mRNA (for example, T7/T3 RNA polymerase mRNA) is mixed with 0.5 mL fresh cell culture medium containing 1~50 µL of In-VivoJetPEI transfection reagent, depending on the desired final mixture concentration. After short incubation, the above mixture is then added into a cell culture condition containing 20%-65% confluency of the cultivated cells of interest, depending on the test design and purpose. Alternatively, for intracellular siRNA transfection, siRNA/shRNA (for example, anti-copGFP) is mixed with 0.5 mL fresh cell culture medium containing 1~50 µL of In-VivoJetPEI transfection reagent and subsequently added into each cell culture, resulting in a final siRNA/shRNA concentration ranged from 1 to 2000 nanomolar (nM), depending on the desired final dosage needed. For long-term maintenance, the cell culture medium is reflashed every 24 to 72 hours, depending on the transfected cell types. This intracellular transfection procedure may be performed repeatedly to increase transfection efficiency. The results after treatments of either siRNA/shRNA or mRNA transfection are shown in FIGs. 1 and 4, respectively.

### 2. In-Vivo Delivery/Transfection

The composition of a designed PLRcD template and DdRP mRNA mixture (weight ratio ranged from about 200:1 to 1:200) is mixed with a proper amount of a delivery/transfection reagent, such as In-VivoJetPEI agent or other similar LNP-based delivery/transfection reagents, following the manufacturer's protocols, and then injected into blood veins or muscles of an animal, depending on the purpose of involved applications. The delivery/transfection reagent is used for mixing, conjugating, encapsulating and/or formulating the designed composition, so as to not only protect the associated DNA/RNA contents from degradation but also facilitate the delivery/transfection of the designed composition into specific target cells of interest in vitro, ex vivo as well as in vivo.

### 3. Preparation of PLRcD Templates and DdRP mRNA

Reverse transcription (RT) of desired RNA/mRNA is performed by adding about 0.01 ng~10 microgram (µg) of isolated RNA/mRNA into a 20~50 µL RT reaction (SuperScript III cDNA RT kit, ThermoFisher Scientific, MA, USA), following the manufacturer's suggestions. Depending on the RNA/mRNA amount, the RT reaction mixture further contains about 0.01~20 nmole RT primer, 0.1∼10 mM each of deoxyribonucleoside triphosphate molecules (dNTPs; dATP, dTTP, dGTP and dCTP) and reverse transcriptase in 1x RT buffer. Then, the RT reaction is incubated at 37~65°C for 1∼3 hours (hr), depending on the length and structural complexity of the desired RNA/mRNA sequence(s), so as to form the complementary DNA (cDNA) of the desired RNA/mRNA which is then used for the next step of PCR. Regarding RT primer designs, for demonstration but not limited to this design, we used 5'-CAGTTCCAAT TGTGAAGATT CTC-3' (SEQ ID NO:16) for RT of a desired RdRp mRNA sequence and used another 5'-CTTGATGACG TTCTCAGTGC-3' (SEQ ID NO: 17) for RT of another microRNA (miRNA)-containing green fluorescent protein (GFP)-coding mRNA (e.g. eGFP-coding mRNA) of interest, respectively.

Next, polymerase chain reaction (PCR) is performed by adding about 0.01 pg-10 µg of the RT-derived cDNAs in a 20~50 µL PCR preparation mixture (High-Fidelity PCR master kit, ThermoFisher Scientific, MA, USA), following the manufacturer's suggestion. Then, the PCR mixture is incubated in twenty to thirty (20-30) cycles of denaturation at 94°C for 30 sec-1 mim, annealing at 50~58°C for 30 sec-1 min, and then extension at 72°C for 1∼3 min, depending on the structure and length of the desired cDNA sequences, respectively. For demonstration but not limited to this example, we used two sets of PCR primer pairs for amplifying two COVID-19 virus *(i.e.* SARS-CoV-2)-associated PLRcD templates, including a pair of 5'-GATATCTAAT ACGACTCACT ATAGGGAGAG GTGCCACCAT GGTACTTGGT AGTT-3' (SEQ ID NO:18) and SEQ ID NO:16 (for amplifying 12.5k-nucleotide (nt) SARS-CoV-2 RdRp/helicase/S protein-coding RNA) and another pair of 5'-GATATCTAAT ACGACTCACT ATAGGGAGAC TAGTGGCCAC CATGTTCTTG TTAACAACT-3' (SEQ ID NO:19) and SEQ ID NO: 16 (for amplifying 2.8k-nt coronaviral S protein-coding mRNA). Moreover, we used another different pair of PCR primers for amplifying miRNA-containing eGFP-mRNA-coding PLRcD templates, including a 5'-primer 5'-GATATCTAAT ACGACTCACT ATAGGGAGAG GTATGGTACT TGGTAGTT-3' (SEQ ID NO:20) and SEQ ID NO: 17. In principle, the design of 5'-primers must comprise at least a consensus promoter sequence for IVT, including but not limited to T7, T3, and/or SP6 pol promoter sequences, for example, 5'-TCTAATACGA CTCACTATAG GGAGA-3' (SEQ ID NO:21).

For RNA *(e.g.* DdRP mRNA) production, since a promoter has been incorporated into the resulting PLRcD templates, a novel IVT reaction is prepared by using a mixture of 0.01 ng~10 µg of the PCR product, 0.1~10 U of helicase (e.g. coronaviral NSP7 and/or NSP13 proteins; optional), NTPs, and RNA polymerase (*i.e.* T7, T3, and/or SP6) in 1x transcription buffer. Then, the IVT reaction is incubated at 37°C for 1∼6 hr, depending on the stability and activity of the used RNA polymerase(s). The resulting mRNA (*e.g.* DdRP mRNA) products are preferably not only 5'-capped by using either vaccina cap enzymes, coronaviral NSP9/14, and/or NSP10/16 proteins, or a combination thereof. Also, the resulting mRNA (e.g. DdRP mRNA) products are preferably further 5'-capped by Preferably, the mRNA of NSP7, NSP13, NSP9/14 and/or NSP10/16, or a combination thereof, can also be used in the mixture composition of the designed PLRcD template and DdRP mRNA for intracellular co-transfection, so as to amplify 5'-capped mRNA in the transfected cells.

### 4. RNA Purification and Northern Blot Analysis

Desired RNAs (10 µg) are isolated with a *mir*Vana^{™} RNA isolation kit (Ambion, Austin, TX) or similar purification filter column, following the manufacturer's protocol, and then further purified by using either 5%~10% TBE-urea polyacrylamide or 1%~3.5% low melting point agarose gel electrophoresis. For Northern blot analysis, the gel-fractionated RNAs are electroblotted onto a nylon membrane. Detection of the RNA and its IVT template (the PCR-derived cDNA product) is performed with a labeled [LNA]-DNA probe complementary to a target sequence of the desired RNA. The probe is further purified by high-performance liquid chromatography (HPLC) and tail-labeled with terminal transferase (20 units) for 20 min in the presence of either a dye-labeled nucleotide analog or [³²P]-dATP (> 3000 Ci/mM, Amersham International, Arlington Heights,IL).

### 5. Protein Extraction and Western Blot Analysis

Cells (10⁶) are lysed with a CelLytic-M lysis/extraction reagent (Sigma) supplemented with protease inhibitors, Leupeptin, TLCK, TAME and PMSF, following the manufacturer's suggestion. Lysates are centrifuged at 12,000 rpm for 20 min at 4°C and the supernatant is recovered. Protein concentrations are measured using an improved SOFTmax protein assay package on an E-max microplate reader (Molecular Devices, CA). Each 30 µg of cell lysate are added to SDS-PAGE sample buffer under reducing (+50 mM DTT) and non-reducing (no DTT) conditions, and boiled for 3 min before loading onto a 6~8% polyacylamide gel. Proteins are resolved by SDS-polyacrylamide gel electrophoresis (PAGE), electroblotted onto a nitrocellulose membrane and incubated in Odyssey blocking reagent (Li-Cor Biosciences, Lincoln, NB) for 2 hr at room temperature. After that, a primary antibody is applied and mixed to the reagent and then together incubated at 4°C. After overnight incubation, the membrane is rinsed three times with TBS-T and then exposed to goat anti-mouse IgG conjugated secondary antibody to Alexa Fluor 680 reactive dye (1:2,000; Invitrogen-Molecular Probes), for 1 hr at the room temperature. After three additional TBS-T rinses, fluorescent scanning of the immunoblot and image analysis are conducted using Li-Cor Odyssey Infrared Imager and Odyssey Software v.10 (Li-Cor).

### 6. Immunostaining Assay

Cell/Tissue samples are first fixed in 100% methanol for 30 min at 4°C and then 4% paraformaldehyde (in 1x PBS, pH 7.4) for 10 min at 20°C. After that, the samples are further incubated in 1x PBS containing 0.1%~0.25% Triton X-100 for 10 min and then washed in 1x PBS three times for 5 min. For immunostaining, corresponding primary antibodies were purchased from Invitrogen (CA, USA) and Sigma-Aldrich (MO, USA), respectively. Dye-labeled goat anti-rabbit or horse anti-mouse antibody are used as the secondary antibody (Invitrogen, CA, USA). Results are examined and analyzed at 100x or 200x magnification under a fluorescent 80i microscopic quantitation system with a Metamorph imaging program (Nikon).

### 7. Statistic Analysis

All data were shown as averages and standard deviations (SD). Mean of each test group was calculated by AVERAGE of Microsoft Excel. SD was performed by STDEV. Statistical analysis of data was performed by One-Way ANOVA. Tukey and Dunnett's t post hoc test were used to identify the significance of data difference in each group. *p* <0.05 was considered significant (SPSS v12.0, Claritas Inc).

### REFERENCES:

1. Boo and Kim; The emerging role of RNA modifications in the regulation of mRNA stability. Exp. Mol. Med. 52:400-408, 2020.
2. Yuan et al.; Human DNA exonuclease TREX1 is also a exoribonuclease that acts on single-stranded RNA. J. Biol. Chem. 290:13344-13353, 2015.
3. McDowell et al.; Determination of intrinsic transcription termination efficiency by RNA polymerase elongation rate. Science 266:822-825, 1994.

## Claims

1. A modified RNA composition comprising at least an oxo-nucleotide containing oxopurine in the 3'-end region of its sequence, wherein said oxo-nucleotide contains 8-hydroxyguanine (8-oxoguanine; oxo-G/oxo-dG) or 8-hydroxyadenine (8-oxoadenine; oxo-A/oxo-dA).

2. The modified RNA composition as defined in Claim 1, wherein the conformation of said modified RNA composition is a single-stranded sequence or double-stranded duplex.

3. The modified RNA composition as defined in Claim 1, wherein the construct of said modified RNA composition contains highly structured conformations.

4. The modified RNA composition as defined in Claim 1, wherein the construct of said modified RNA composition is designed to mimic non-coding RNA (ncRNA).

5. The modified RNA composition as defined in Claim 4, wherein the non-coding RNA is single-stranded RNA selected from antisense RNA oligonucleotide (aRNA-ASO), short hairpin RNA (shRNA), microRNA precursor (pre-miRNA), and long noncoding RNA (lncRNA), or a combination thereof.

6. The modified RNA composition as defined in Claim 4, wherein the non-coding RNA is double-stranded duplex RNA selected from small interfering RNA (siRNA), microRNA (miRNA) mimic, RNA-DNA hybrid, and small activating RNA (saRNA), or a combination thereof.

7. The modified RNA composition as defined in Claim 1, wherein the construct of said modified RNA composition is designed to mimic protein-/peptide-coding mRNA.

8. The modified RNA composition as defined in Claim 7, wherein said protein-/peptide-coding mRNA is self-amplifying RNA/mRNA (saRNA/samRNA).

9. The modified RNA composition as defined in Claim 7, wherein said protein-/peptide-coding mRNA is capped by a 5'-cap molecule.

10. The modified RNA composition as defined in Claim 7, wherein said protein-/peptide-coding mRNA contains a poly-A tail.

11. The modified RNA composition as defined in Claim 1, wherein U (uridine/uracil) content of said modified RNA composition is further completely or partially replaced by another modified nucleotide analog.

12. The modified RNA composition as defined in Claim 11, wherein said another modified nucleotide analog is pseudouridine (p), methyluridine (t/tm/um), methylpseudouridine (fm/m1f), or methoxyuridine (mo5U), or a combination thereof.

13. The modified RNA composition as defined in Claim 1, wherein C (cytosine/cytidine) content of said modified RNA composition is further completely or partially replaced by another modified nucleotide analog.

14. The modified RNA composition as defined in Claim 13, wherein said another modified nucleotide analog is 5-methylcytidine (m5C) or N4-acetylcytidine (ac4C).

15. The modified RNA composition as defined in Claim 1, wherein said modified RNA composition is used to prevent TREX1-mediated degradation and related non-specific immunity over-activation.

16. The modified RNA composition as defined in Claim 1, wherein said modified RNA composition is a pharmaceutical compound or composition.

17. The modified RNA composition as defined in Claim 1, wherein said modified RNA composition is further formulated with at least a delivery agent for facilitating intracellular transfection in vitro, ex vivo as well as in vivo.

18. The modified RNA composition as defined in Claim 17, wherein said delivery agent is liposomes, nanoparticles, liposomal nanoparticles (LNP), exosomes, conjugating molecules, infusion/transfusion agents, triglycylglycerin (TGG)-derived molecules, electroporation agents, or transposons/retrotransposons, or a combination thereof.

19. The modified RNA composition as defined in Claim 1, wherein said modified RNA composition is made by using an oligonucleotide synthesizer machine.

20. The modified RNA composition as defined in Claim 1, wherein said modified RNA composition is made by an enzymatic reaction using terminal deoxynucleotidyl transferases (TdT), terminal uridylyl transferases, or polymerase theta.


SEQUENCE LISTING

(1) GENERAL INFORMATION:
(iii) NUMBER OF SEQUENCES: 24

(2) INFORMATION FOR SEQ ID NO:1:
(i) SEQUENCE CHARACTERISTICS:
(A) LENGTH: 6 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:1:
RCCRCC 6

(2) INFORMATION FOR SEQ ID NO:2:
(i) SEQUENCE CHARACTERISTICS:
(A) LENGTH: 6 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:2:
RCCDCC 6

(2) INFORMATION FOR SEQ ID NO:3:
(i) SEQUENCE CHARACTERISTICS:
(A) LENGTH: 73 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:3:
GCUCCCUUCA ACUUUAACAU GGAAGUGCUU UCUGUGACUU
UAAAAGUAAG UGCUUCCAUG UUUUAGUAGG AGU 73

(2) INFORMATION FOR SEQ ID NO:4:
(i) SEQUENCE CHARACTERISTICS:
(A) LENGTH: 68 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:4:
CCUUUGCUUU AACAUGGGGG UACCUGCUGU GUGAAACAAA
AGUAAGUGCU UCCAUGUUUC AGUGGAGG 68

(2) INFORMATION FOR SEQ ID NO:5:
(i) SEQUENCE CHARACTERISTICS:
(A) LENGTH: 69 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:5:
CCACCACUUA AACGUGGAUG UACUUGCUUU GAAACUAAAG
AAGUAAGUGC UUCCAUGUUU UGGUGAUGG 69

(2) INFORMATION FOR SEQ ID NO:6:
(i) SEQUENCE CHARACTERISTICS:
(A) LENGTH: 68 base pairs
(B) TYPE: nucleic acid
(C) STRANDEDNESS: single
(D) TOPOLOGY: hairpin

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:6:
CCUCUACUUU AACAUGGAGG CACUUGCUGU GACAUGACAA
AAAUAAGUGC UUCCAUGUUU GAGUGUGG 68

(2) INFORMATION FOR SEQ ID NO:7:
(i) SEQUENCE CHARACTERISTICS:
(A) LENGTH: 44 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:7:
CUGUGUGGCU GUCACUCGGC UGCAUGCUUA GUGCACUCAC GCAG
44

(2) INFORMATION FOR SEQ ID NO:8:
(i) SEQUENCE CHARACTERISTICS:
(A) LENGTH: 65 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:8:
CUGUGUGGCU GUCACUCGGC UGCAUGCUUA GUGCACUCAC
GCAGUAUAAU UAAUAACUAA UUACU 65

(2) INFORMATION FOR SEQ ID NO:9:
(i) SEQUENCE CHARACTERISTICS:
(A) LENGTH: 147 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:9:
GUCGUUGACA GGACACGAGU AACUCGUCUA UCUUCUGCAG
GCUGCUUACG GUUUCGUCCG UGUUGCAGCC GAUCAUCAGC
ACAUCUAGGU UUCGUCCGGG UGUGACCGAA AGGUAAGAUG
GAGAGCCUUG UCCCUGGUUU CAACGAG 147

(2) INFORMATION FOR SEQ ID NO:10:
(i) SEQUENCE CHARACTERISTICS:
(A) LENGTH: 88 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:10:
AAUUAUAAAU UACCAGAUGA UUUUACAGGC UGCGUUAUAG
CUUGGAAUUC UAACAAUCUU GAUUCUAAGG UUGGUGGUAA
UUAUAAUU 88

(2) INFORMATION FOR SEQ ID NO:11:

(i) SEQUENCE CHARACTERISTICS:
(A) LENGTH: 129 base pairs
(B) TYPE: nucleic acid
(C) STRANDEDNESS: single
(D) TOPOLOGY: multiple stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:11:
CACAAAUAUU ACCAGAUCCA UCAAAACCAA GCAAGAGGUC
AUUUAUUGAA GAUCUACUUU UCAACAAAGU GACACUUGCA
GAUGCUGGCU UCAUCAAACA AUAUGGUGAU UGCCUUGGUG
AUAUUGCUG 129

(2) INFORMATION FOR SEQ ID NO:12:
(i) SEQUENCE CHARACTERISTICS:
(A) LENGTH: 189 base pairs
(B) TYPE: nucleic acid
(C) STRANDEDNESS: single
(D) TOPOLOGY: multiple stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:12:
GCAAAAAUGU GAUCUUGCUU GUAAAUACAA UUUUGAGAGG
UUAAUAAAUU ACAAGUAGUG CUAUUUUUGU AUUUAGGUUA
GCUAUUUAGC UUUACGUUCC AGGAUGCCUA GUGGCAGCCC
CACAAUAUCC AGGAAGCCCU CUCUGCGGUU UUUCAGAUUC
GUUAGUCGAA AAACCUAAGA AAUUUAAUG 189

(2) INFORMATION FOR SEQ ID NO:13:
(i) SEQUENCE CHARACTERISTICS:
(A) LENGTH: 294 base pairs
(B) TYPE: nucleic acid
(C) STRANDEDNESS: single
(D) TOPOLOGY: multiple stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:13:
CACUCCCCUG UGAGGACUAC UGUCUUCACG CAGAAAGCGU
CUAGCCAUGG CGUUAGUAUG AGUGUCGUGC AGCCUCCAGG
ACCCCCCCUC CCGGGAGAGC CAUAGUGGUC UGCGGAACCG
GUGAGUACAC CGGAAUUGCC AGGACGACCG GGUCCUUUCU
UGGAUCAACC CGCUCAAUGC CUGGAGAUUU GGGCGUGCCC
CCGCGAGACU GCUAGCCGAG UAGUGUUGGG UCGCGAAAGG
CCUUGUGGUA CUGCCUGAUG GGUGCUUGCG AGUGCCCCGG
GAGGUCUCGU AGAC 294

(2) INFORMATION FOR SEQ ID NO:14:
(i) SEQUENCE CHARACTERISTICS:
(A) LENGTH: 55 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:14:
GGACACGAGU AACUCGUCUA UCUUCUGCAG GCUGCUUACG
GUUUCGUCCG UGUUG 55

(2) INFORMATION FOR SEQ ID NO:15:
(i) SEQUENCE CHARACTERISTICS:
(A) LENGTH: 52 base pairs
(B) TYPE: nucleic acid
(C) STRANDEDNESS: single
(D) TOPOLOGY: stem-loop

(ii) MOLECULE TYPE: RNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:15:
CAGCCGAUCA UCAGCACAUC UAGGUUUUGU CCGGGUGUGA
CCGAAAGGUA AG 52

(2) INFORMATION FOR SEQ ID NO:16:
(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: YES

(xi) SEQUENCE DESCRIPTION: SEQ ID NO:16:
CAGTTCCAAT TGTGAAGATT CTC 23

(2) INFORMATION FOR SEQ ID NO:17:
(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: YES

(xi) SEQUENCE DESCRIPTION: SEQ ID NO:17:
CTTGATGACG TTCTCAGTGC 20

(2) INFORMATION FOR SEQ ID NO:18:
(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:18:
GATATCTAAT ACGACTCACT ATAGGGAGAG GTGCCACCAT
GGTACTTGGT AGTT 54

(2) INFORMATION FOR SEQ ID NO:19:
(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:19:
GATATCTAAT ACGACTCACT ATAGGGAGAC TAGTGGCCAC
CATGTTCTTG TTAACAACT 59

(2) INFORMATION FOR SEQ ID NO:20:
(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:20:
GATATCTAAT ACGACTCACT ATAGGGAGAG GTATGGTACT
TGGTAGTT 48

(2) INFORMATION FOR SEQ ID NO:21:
(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: DNA
(A) DESCRIPTION: /desc = "synthetic"

(iii) HYPOTHETICAL: NO

(iv) ANTI-SENSE: NO

(xi) SEQUENCE DESCRIPTION: SEQ ID NO:21:
TCTAATACGA CTCACTATAG GGAGA 25

(2) INFORMATION FOR SEQ ID NO:22:
(i) SEQUENCE CHARACTERISTICS:
(A) LENGTH: 26 base pairs
(B) TYPE: nucleic acid
(C) STRANDEDNESS: duplex
(D) TOPOLOGY: linear

(ii) MOLECULE TYPE: RNA
(A) DESCRIPTION: /desc = "synthetic"

(iii) HYPOTHETICAL: NO

(iv) ANTI-SENSE: YES

(xi) SEQUENCE DESCRIPTION: SEQ ID NO:22:
CUCUUGAAGU GCAUGUGGCU GUCTT*dG 26

(2) INFORMATION FOR SEQ ID NO:23:
(i) SEQUENCE CHARACTERISTICS:
(A) LENGTH: 25 base pairs
(B) TYPE: nucleic acid
(C) STRANDEDNESS: duplex
(D) TOPOLOGY: linear

(ii) MOLECULE TYPE: RNA
(A) DESCRIPTION: /desc = "synthetic"

(iii) HYPOTHETICAL: NO

(iv) ANTI-SENSE: YES

(xi) SEQUENCE DESCRIPTION: SEQ ID NO:23:
CUCUUGAAGU GCAUGUGGCU GUCTT 25

(2) INFORMATION FOR SEQ ID NO:24:
(i) SEQUENCE CHARACTERISTICS:
(A) LENGTH: 21 base pairs
(B) TYPE: nucleic acid
(C) STRANDEDNESS: duplex
(D) TOPOLOGY: linear

(ii) MOLECULE TYPE: RNA
(A) DESCRIPTION: /desc = "synthetic"

(iii) HYPOTHETICAL: YES

(iv) ANTI-SENSE: YES

(xi) SEQUENCE DESCRIPTION: SEQ ID NO:24:
UUCUCCGAAC GUGUCACGUT T 21